# FASCICULE DE BREVET EUROPEEN

(11) **EP 0 818 184 B1**
(45) Date de publication et mention de la délivrance du brevet: **10.12.2003**
(21) Numéro de dépôt: 97401545.5
(22) Date de dépôt: 01.07.1997
(51) Int. Cl.: A61F 2/06

(54) **Prothèse médicale en particulier pour anévrismes à liaison entre sa gaine et sa structure**
Prothese, insbesondere bei Aneurismus mit einem Anschluss zwischen ihrem Gewebe und ihrer Struktur
Medical prosthesis, especially for aneurysms, with a connection between its liner and its structure

(30) Priorité: 10.07.1996 FR 9608610
(43) Date de publication de la demande: 14.01.1998
(73) Titulaire: B. BRAUN CELSA, 86360 Chasseneuil du Poitou (FR)
(72) Inventeur: Cottenceau, Jean-Philippe, 92160 Antony (FR); Nadal, Guy, 86000 Poitiers (FR); Chevillon, Gérard, 92120 Montrouge (FR); Roussigne, Maurice, 86000 Poitiers (FR)
(74) Mandataire: Lerner, François

(56) Documents cités:
- EP-A- 0 686 379
- FR-A- 2 722 678

## Description

L'invention concerne une prothèse médicale adaptée pour être implantée dans un conduit, ou à la réunion de plusieurs conduits, d'un corps vivant

L'invention trouve en particulier son application pour le traitement de certaines altérations vasculaires, et tout particulièrement les anévrismes.

En particulier dans ce domaine des prothèses vasculaires (qui sont souvent implantables par voie endoluminale, mais qui dans d'autres cas peuvent être implantées par dénudation de vaisseaux), il existe déjà des prothèses comprenant :
- une armature définie par un ou plusieurs tronçons tubulaires suivant au moins un axe de tube, le(au moins certains des) tronçon(s) comportant une structure à méandres qui a une rigidité et présente des sommets entre lesquels s'étendent des segments allongés, les méandres du(des) tronçon(s) tubulaire(s) étant enroulés, suivant un ou plusieurs niveaux d'enroulement,
- une gaine souple sensiblement coaxiale à l'armature, pour y canaliser un fluide circulant dans le conduit dudit corps,
- et des moyens de liaison entre la gaine et l'armature, ces moyens de liaison comprenant au moins un fil souple de rigidité inférieure à celle desdits segments de la structure, ledit fil souple étant disposé, pour assurer cette liaison, de manière qu'il chevauche au moins un segment et passe dans la gaine,

Un exemple de telles prothèses existe dans EP-A-686 379.

L'armature de telles prothèses est maintenant souvent dénommée « stents », les stents désignant également ces prothèses lorsqu'elles sont dépourvues de gaine.

D'autres exemples de stents peuvent être trouvés dans EP-A-540 290,WO-A-95/26 695 ou WO-A-94/17 754 qui présentent une armature à plusieurs niveaux d'enroulement sensiblement coaxiaux formés individuellement par une structure à méandres comprenant des segments allongés ayant une certaine rigidité, les tronçons étant reliés entre eux successivement, deux à deux, par au moins l'un desdits segments de structure.

Dans WO-A-95/21 592, il a en outre été imaginé d'appliquer une armature construite comme ci-avant, à la réalisation d'une prothèse « bifurquée », c'est-à-dire dont l'armature comprend un tronçon tubulaire principal et deux tronçons tubulaires secondaires réunis entre eux et au tronçon tubulaire principal à l'endroit d'une zone de jonction sensiblement en « Y ».

Sur les prothèses existantes, lorsqu'elles comportent une gaine de canalisation de fluide (comme dans EP-A-539 237 ou WO-A-95/26 695), certaines imperfections ont été notées, quant à la manière dont la liaison entre l'armature (ou la partie « stent ») et la gaine est réalisée.

Ainsi, dans EP-A-539 237, cette liaison est assurée par des fils de suture en matériau non biodégradable qui forment localement de petits liens qui passent à travers la gaine et sont noués autour d'un segment de structure de l'armature.

Il ne s'agit là toutefois que d'une liaison ponctuelle, assez longue à réaliser, la distance entre deux liens s'avérant en pratique assez importante, ce qui peut s'avérer dommageable dans l'éventualité notamment où un lien se dénouerait ou serait rompu, laissant alors « flotter » la gaine.

Pour résoudre ce problème de fixation optimisée de la gaine à l'armature (que celle-ci soit située autour ou à l'intérieur, au contact, de la gaine) la solution de l'invention est de prévoir que :
- l'armature présente plusieurs tronçons tubulaires et/ou plusieurs niveaux d'enroulement,
- et le (au moins l'un des) fil(s) souple(s) s'étend successivement, ainsi disposé, le long d'au moins deux segments de structure appartenant à des niveaux d'enroulement différents et/ou à des tronçons tubulaires différents.

Outre la rapidité possible de réalisation de cet assemblage (notamment en utilisant un « point lancé » avec un enroulement en hélice des fils), on va accroître « l'intimité » de la liaison entre la gaine et la structure.

En outre, il s'est avéré que la solution de l'invention résout implicitement un problème parfois gênant lié à la saillie que peuvent faire les apex des méandres de la structure, lorsque cette dernière est courbée, par exemple lors de son implantation dans son conduit récepteur.

Dans ce cas, il est de préférence prévu que le fil souple passe, au passage d'un segment à l'autre, à proximité ou autour d'un de leurs sommets pour favoriser la retenue de ce sommet lorsque l'on courbe la prothèse à cet endroit.

Selon encore d'autres caractéristiques de l'invention, il sera avantageusement prévu :
- que le fil souple considéré soit enroulé essentiellement en hélice autour desdits segments, suivant un point lancé,
- et/ou que ces segments soient situés sensiblement dans la continuité l'un de l'autre, suivant une direction sensiblement rectiligne ou en hélice, le fil souple considéré s'étendant ainsi de manière générale suivant cette direction.

Pour une efficacité de mise en place des liens souples, les fils constituant ces liens passeront de préférence d'un premier niveau à un second niveau de la structure, sans revenir au premier niveau.

Et pour limiter les risques de laisser « filer » librement le fil en cas de rupture inopinée, le fil souple considéré sera noué au moins une fois sur sa longueur (et de préférence plusieurs fois, d'endroits en endroits).

Selon une caractéristique complémentaire, le fil en question passera de préférence dans la gaine après chaque chevauchement du segment de structure considéré.

A noter également qu'au moins certains des fils souples de liaison utilisés s'étendront de préférence sur au moins l'essentiel de la longueur de la prothèse (cette longueur étant mesurée entre les extrémités axiales libres de ladite prothèse).

Une description plus détaillée de l'invention va maintenant être donnée en relation avec les dessins d'accompagnement dans lesquels :
Les figures 1, 2, 3, 4, 5 et 6 sont des réalisations d'armature conformes à l'art antérieur ;
La figure 7 reprend la structure de la figure 1, avec adjonction des fils souples de maintien caractéristiques de l'invention, sur une prothèse pour anévrisme ;
La figure 8 est une vue agrandie du détail repéré VIII sur les figures 1 ou 3, avec adjonction des fils de maintien caractéristiques de l'invention et de la gaine,
La figure 9 est une vue agrandie du détail IX de la figure 8,
La figure 10 est une vue de détail comparable à celle de la figure 8, mais représentant une variante de réalisation, et
Les figures 11 et 12 reprennent les structures des figures 5 et 6, avec adjonction des fils souples de maintien de liaison gaine/structure propres à l'invention, et de la gaine elle-même.

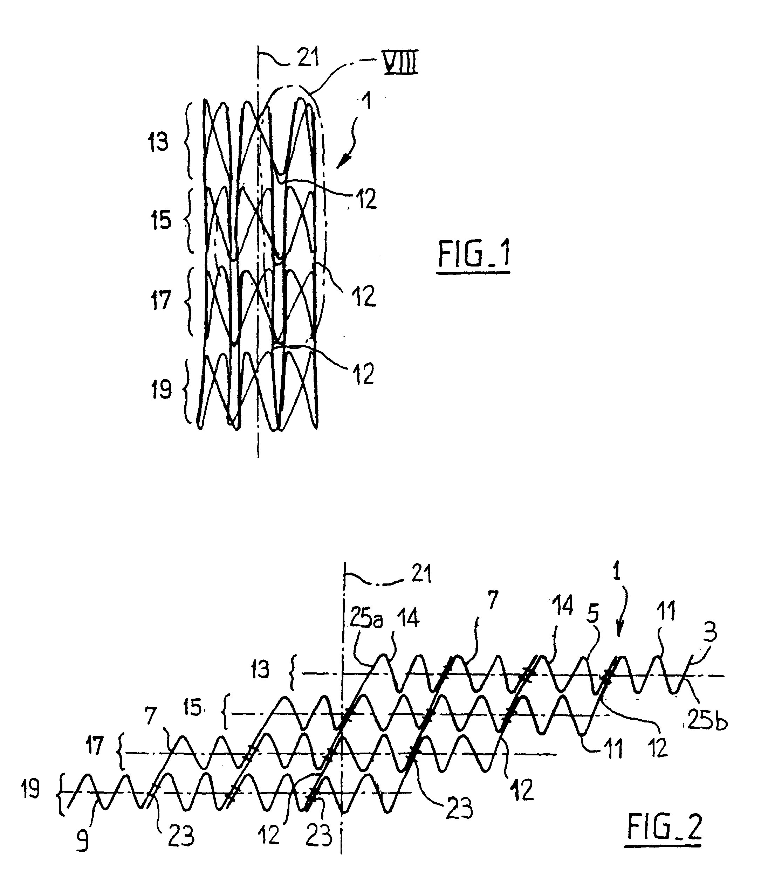

Sur les figures 1 et 2, on voit représenté un stent constitué à partir de plusieurs fils métalliques ondulés 3, 5, 7, 9 (repérés sur la figure 2).

Il s'agit de fils relativement rigides, par exemple en acier inoxydable de quelque dixièmes de millimètre de diamètre.

Un ou plusieurs fils en « nitinol » (marque déposée) auraient également pu convenir.

Pour réaliser l'armature 1 constituant ce stent, on peut procéder comme montré sur la figure 2 : on part de quatre fils ronds 3, 5, 7, 9 que l'on conforme avec des ondulations sensiblement « en V » à sommets 11 arrondis, de telle sorte que par exemple il y ait, tous les quatre sommets, un segment rectiligne 12 de longueur sensiblement double des autres segments rectilignes 14, et ce avec une juxtaposition des fils 3, 5, 7, 9 suivant une série de lignes directrices dirigée sensiblement perpendiculairement à l'axe 21 prévu pour la prothèse et des points de soudure 23 assurant la liaison deux à deux des fils métalliques, à chaque fois que deux fils différents sont juxtaposés bord à bord à un même niveau d'ondulation, tels que 13,15,17, 19. Sur la figure 2, de courts tirets en biais schématisent ces points de soudure.

Après avoir réalisé ainsi une structure « à plat », il suffit d'enrouler la structure sur elle-même, à chaque niveau, en soudant à chaque fois les segments d'ondulations situés en bout de fil tels que ceux repérés 25a et 25b pour le niveau 13.

On obtient ainsi la structure étagée de la figure 1 constituée des quatre tronçons tubulaires 13,15,17,19 formés individuellement par lesdits fils en zigzag, les segments 12 de fil de plus grande longueur assurant la jonction entre deux niveaux de tubes en zigzag successifs.

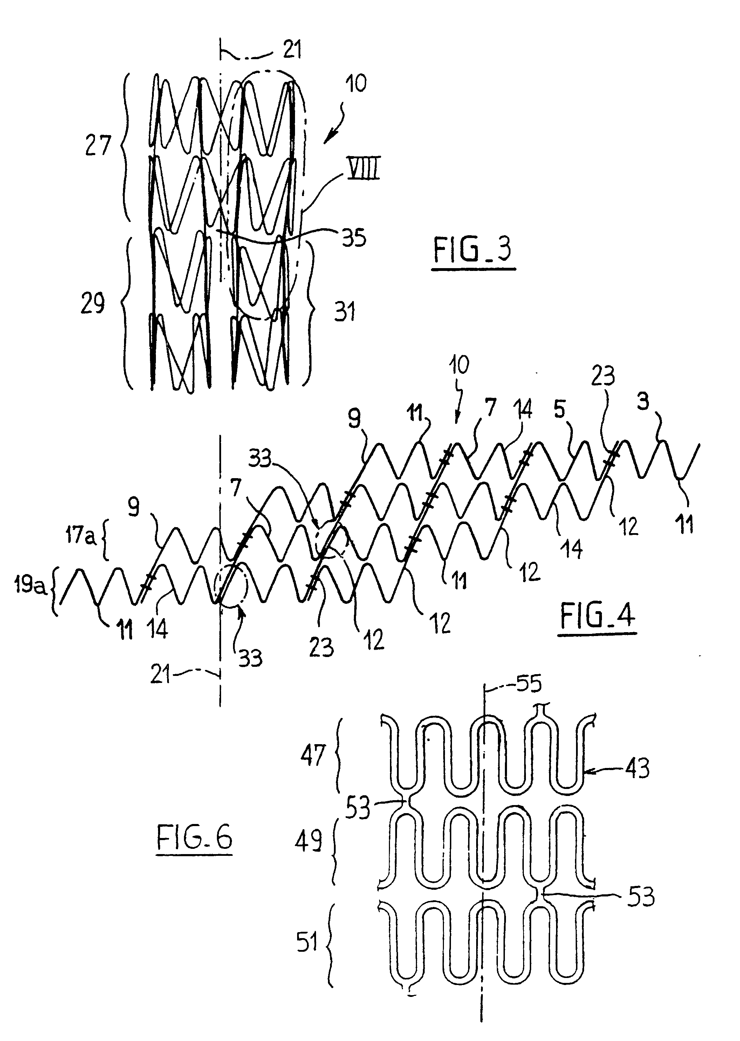

Sur la figure 3, il s'agit d'un stent bifurqué 10 comprenant un tronçon (ou une branche) principal(e) 27 auquel sont raccordées deux branches ou tronçons secondaires 29, 31.

Comme on peut le voir sur la figure 4, la réalisation du stent bifurqué 10 de la figure 3 est pratiquement identique à celle du stent « monotube » de la figure 1.

En effet, on retrouve sur la figure 4 la structure de la figure 2, sauf aux endroits repérés 33 sur la figure 4 où deux segments sensiblement rectilignes de fils structuraux ne sont pas soudés ensemble, bien que disposés bord à bord.

C'est grâce à cette absence de liaison intime entre les fils 5 et 7 aux niveaux repérés 17a et 19a que l'on réussit à créer les deux branches 29 et 31, à partir de la zone de bifurcation repérée 35.

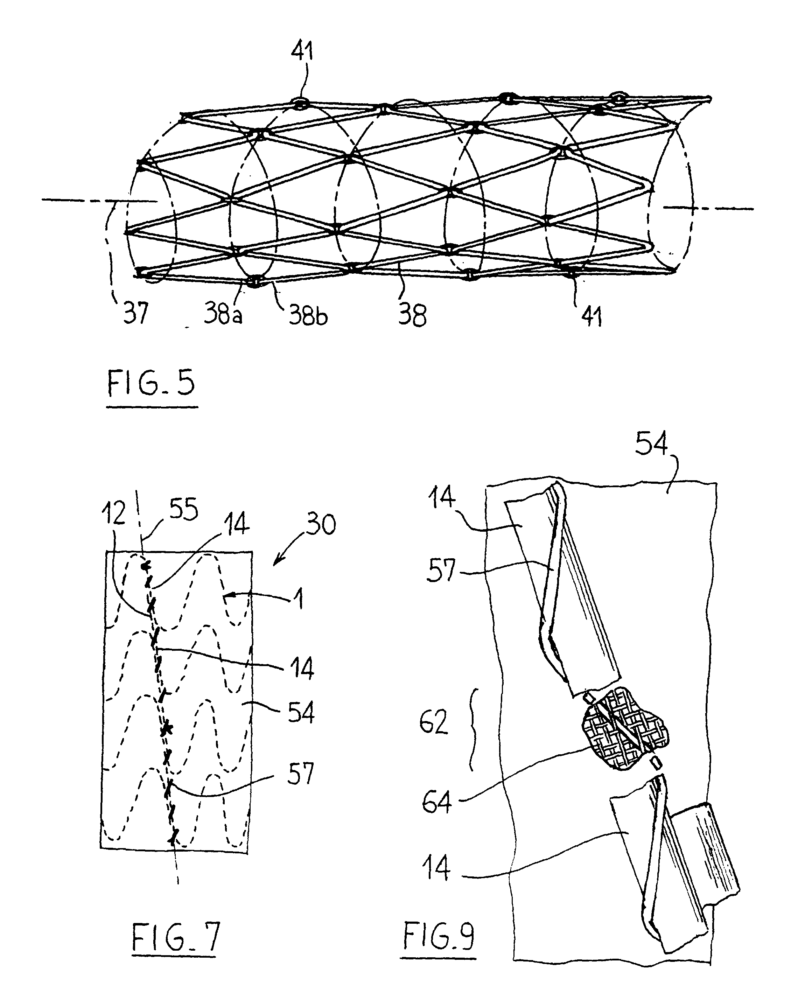

Sur la figure 5, on retrouve une prothèse « monotube », à axe de tube unique 37. Cette prothèse est conforme à la description qui en est donnée dans US-A-5 405 377.

Brièvement, il s'agit d'un stent constitué à partir d'un unique fil structurel 38 relativement rigide, tel qu'un fil métallique (par exemple acier inoxydable, tentale, titane) ou en une matière plastique, suffisamment rigide, résistant et élastique pour avoir une configuration sinueuse tout en étant ensuite enroulé pour définir une hélice continue avec une série de spirales reliées entre elles par de petits anneaux 41 (par exemple métalliques) reliant tous les apex adjacents 38a, 38b des zigzags formés par le fil 38.

Une autre structure de l'art antérieur connue est illustrée figure 6.

Il s'agit d'une des structures décrites dans EP-A-540 290.

La structure montrée localement sur la figure 6, consiste en un stent tubulaire de section circulaire constante. Sa structure ou armature peut être réalisée à partir d'une fine plaque métallique, par érosion chimique.

On peut ainsi former une structure 43 présentant une série de sinuosités, deux niveaux de sinuosités, tels que 47 et 49 ou 49 et 51, étant reliés par des ponts de matière 53 dirigés parallèlement à l'axe de tube 55 de la structure.

EP-A-540 290 (parmi d'autres publications) enseigne que pour implanter un stent on peut utiliser un ballon que l'on gonfle dans le conduit d'implantation et qui permet de dilater de manière plastique le stent afin de le plaquer contre la paroi dudit conduit.

Les figures 7 et suivantes ont pour objet de montrer que la solution de l'invention s'applique à de nombreux types de prothèses pour anévrismes et plus généralement de stents « habillés » par une gaine 54 de canalisation du fluide circulant dans le conduit d'implantation considéré (sang en particulier), les stents illustrés sur les figures précédentes 1 à 6 étant en particulier concernés.

Ainsi sur la figure 7, on retrouve le stent de la figure 1, les figures 8 et 9 montrant deux vues de détails agrandis, plus lisibles.

Ainsi on peut constater qu'aux différents étages de la structure, chacune des lignes 55 formées par les segments de liaison 12 de double longueur liés chacun intimement au segment 14 de simple longueur des zigzags correspondants, est entourée par un fil ou filament souple 57 qui traverse au passage la gaine 54.

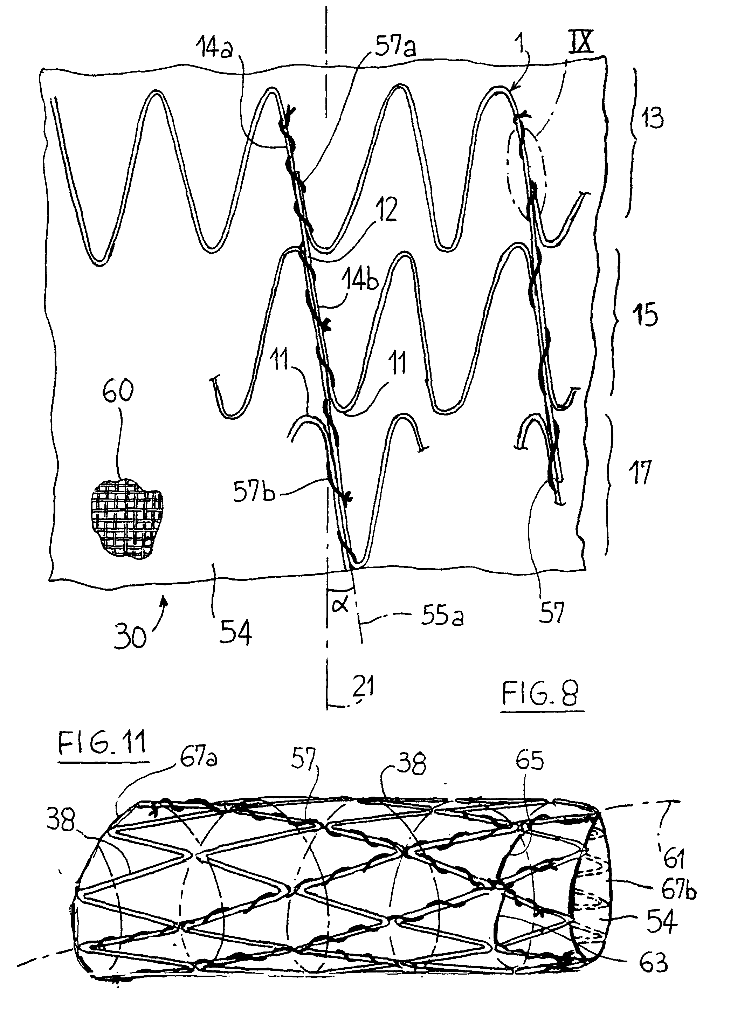

Ainsi, chaque fil souple 57 entoure un premier segment intermédiaire, tel que 14a sur la figure 8, puis passe de ce segment à un autre 14b appartenant à un autre étage d'enroulement, le fil 57 s'enroulant de nouveau le long du second segment 14b, et ainsi de suite, en l'espèce sur toute la longueur de la ligne repérée 55a, laquelle présente comme les autres lignes 55, une direction sensiblement rectiligne (ou légèrement en hélice) angulairement proche de l'axe 21 du stent (angle inférieur à 40 degrés environ, et plus généralement compris entre environ 3 et 25 degrés).

Les fils 57 pourront être en nylon (marque déposée), ou plus généralement en toute fibre naturelle ou synthétique plus souple que les éléments (3, 5, 7, 9, 38, 43...) constituant les structures des stents, tout en étant résistante et bio-compatible. En pratique, tout fil pouvant former un lien en chirurgie peut convenir. Eventuellement, un très fin fil métallique d'environ 0,02 mm à 0,04 mm de diamètre pourrait même être utilisé.

En même temps qu'ils serpentent le long des segments de la structure 1, les filaments 57 passent également (de préférence à chaque tour) à travers la gaine tubulaire 54 qui ici entoure l'armature, pour constituer avec cette armature une prothèse pour anévrisme, 30.

Sur les figures 8,10 et 11, on notera que chaque fil 57 est enroulé suivant un « point lancé », c'est-à-dire enroulé en spirale, l'axe de cette spirale étant sensiblement rectiligne, les fils 57 ne se chevauchant pas eux-mêmes sur leur longueur et ne revenant pas en arrière lorsqu'ils passent d'un niveau d'enroulement (13,15, sur la figure 8) au suivant (15,17).

Sur une même ligne, plusieurs fils peuvent être utilisés à la suite les uns des autres, tels par exemple que les fils 57a et 57b sur la figure 8, les deux extrémités de chaque fil étant nouées autour des fils plus rigides de structure correspondants.

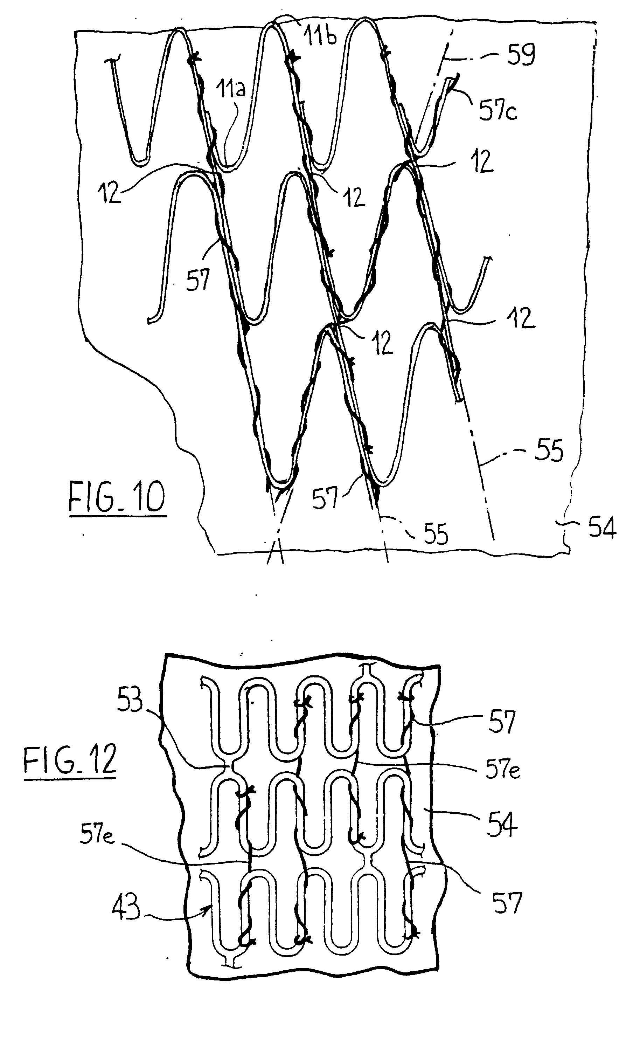

Sur la figure 8, la zone en arrachée marquée 60 schématise le fait que la gaine 54 est à structure maillée. Avec les arrachements de la vue agrandie de la figure 9, on peut voir que le fil 57, lorsqu'il passe derrière un segment structurel 14 (tel que sur sa portion 62), pénètre dans les mailles 64 de la gaine pour s'y entrelacer, avant d'en ressortir pour à nouveau chevaucher le segment structurel 14.

En enroulant ainsi chaque fil de liaison 57, 57a, 57b... sur une longueur suffisante le long des segments structuraux qu'il chevauche, le fil correspondant va implicitement avoir une action de maintien de l'apex 11 à proximité (ou éventuellement autour) duquel il passe, en évitant ainsi que lors d'une courbure de la prothèse, l'apex 11 en question fasse (exagérément) saillie (voir figure 8).

Sur la figure 10, la structure en fils multiples de la figure 8 a été modifiée légèrement pour qu'il y ait un segment 12 de changement d'étage tous les deux apex (tels que 11a, 11b).

Quant aux filaments souples de maintien 57, un fil supplémentaire 57c a été figuré, pour montrer que l'on peut tout à fait croiser ces filaments, en les disposant d'endroits en endroits (voire systématiquement) le long non seulement des lignes 55, mais également de tout ou partie des segments structuraux définissant les lignes fictives rectilignes 59 qui les croisent, d'étage en étage, compte-tenu de la forme « en V » des fils structuraux, tout en passant bien entendu dans la gaine 54.

La figure 11 qui reprend la structure de stent de la figure 5 montre que la technique de liaison filamentaire par les fils souples 57 entre la gaine et le stent telle que prévue dans l'invention peut s'appliquer aussi à un stent à fil structurel enroulé en zigzag et en hélice, sans que les apex de ces fils structuraux soient nécessairement liés entre eux.

Sur la figure 11, la gaine 54 est située à l'intérieur du stent. Les fils souples 57 s'enroulent pour certains suivant une ligne sensiblement rectiligne ou plus ou moins en légère hélice, telle que suivant la direction 61, ou encore avec un décalage angulaire à certains changements de niveau, comme en 63 et 65, tout en s'étendant entre les deux extrémités libres axiales 67a, 67b de la prothèse, sur toute sa longueur.

Sur la figure 12, on retrouve la structure de la figure 6 pour noter qu'ont été adjoints, outre la gaine 54, une série de filaments souples 57 conformes à l'invention qui s'enroulent chacun autour d'un segment d'ondulation à un étage donné de la structure, en traversant à chaque tour quelques fibres ou mailles de la gaine, avant de passer d'un apex à un apex d'un étage adjacent, pour à nouveau serpenter autour d'un segment de cet autre étage, et ainsi de suite d'étage en étage, toujours en passant à travers la gaine, périodiquement

Comme précédemment, certains filaments souples de liaison, tels que 57e, peuvent ne serpenter qu'autour de deux segments successifs appartenant à deux étages différents et autour desquels les deux extrémités opposées du filament 57e auront été fixées, par exemple nouées.

A ce sujet, on notera que de préférence les filaments souples seront, sur leur longueur, périodiquement noués avec un noeud solide, pour éviter qu'en cas de rupture inopinée en un endroit de sa longueur, le fil considéré se défasse sur toute sa longueur.

Quant à la gaine 54, elle pourra être réalisée en tissu ou en matériau synthétique, tel que du « nylon » (marque déposée) ou du « dacron » (marque déposée) et présentera une souplesse comparable à un fin tissu.

A partir de ce qui précède, on aura compris que l'invention peut s'appliquer à des types différents de stents ou d'armatures.

Ainsi, le stent ou l'armature de la prothèse pourra comprendre plusieurs tronçons tubulaires indépendants, axialement étagés, et à structure en méandres (zigzag ou créneaux par exemple) ayant chacun un ou plusieurs niveaux d'enroulement, les tronçons n'étant pas structurellement reliés les uns aux autres, de manière à ce qu'il n'y ait pas transmission d'effort entre eux. La réalisation d'une telle prothèse peut, par exemple, s'obtenir par sectionnement des tronçons de liaison 12 de la figure 2,à un étage intermédiaire donné, créant ainsi la discontinuité nécessaire à l'obtention des tronçons successifs.

On obtiendra dans ce cas une prothèse présentant une capacité accrue de courbure, la gaine souple assurant la cohésion entre les différents tronçons séparés de stent (via les fils souples de liaison entre ces stents et ladite gaine).

## Revendications

1. Prothèse médicale adaptée pour être implantée dans un conduit d'un corps vivant et comprenant :
- une armature (1,10) définie par un ou plusieurs tronçons tubulaires (13, 15, 17, 19 ; 27, 29, 31) suivant au moins un axe de tube (21, 55, 37), le ou au moins certains des tronçons comportant une structure à méandres qui a une rigidité et présente des sommets (11) entre lesquels s'étendent des segments allongés (12,14), les méandres du ou des tronçons tubulaires étant enroulés, suivant un ou plusieurs niveaux d'enroulement (17a, 19a ; 47, 49, 51),
- une gaine souple (54) sensiblement coaxiale à l'armature, pour y canaliser un fluide circulant dans le conduit dudit corps,
- et des moyens (57, 57a, 57b, ..., 57e) de liaison entre la gaine et l'armature, ces moyens de liaison comprenant au moins un fil souple de rigidité inférieure à celle desdits segments de la structure, ledit fil souple (57, 57a, ..., 57e) étant disposé, pour assurer cette liaison, de manière qu'il chevauche au moins un segment et passe dans la gaine,
- l'armature présentent plusieurs tronçons tubulaires (13, 15, ...),
**caractérisée en ce que** :
- et le au moins l'un des fils souples s'étend successivement, ainsi disposé, le long d'au moins deux segments de structure (14; 14a, 14b, 12) appartenant à des tronçons tubulaires différents.

2. Prothèse selon la revendication 1, **caractérisée en ce que** le fil souple passe, au passage d'un segment à l'autre, à proximité ou autour d'un de leurs sommets (11) pour favoriser la retenue de ce sommet lorsque l'on courbe la prothèse à cet endroit

3. Prothèse selon la revendication 1 ou la revendication 2, caractérisée ce que le fil souple considéré est enroulé essentiellement en hélice autour desdits au moins deux segments, suivant un point lancé.

4. Prothèse selon la revendication 2 ou la revendication 3, **caractérisée en ce que** lesdits au moins deux segments sont situés sensiblement dans la continuité l'un de l'autre, suivant une direction sensiblement rectiligne ou en hélice, le fil souple considéré s'étendant ainsi de manière générale suivant cette direction.

5. Prothèse selon l'une quelconque des revendications précédentes, **caractérisée en ce qu'**elle comprend plusieurs fils souples (57, 57c, ...) qui s'étendent suivant plusieurs directions sensiblement parallèles entre elles.

6. Prothèse selon l'une quelconque des revendications précédentes **caractérisée en ce que** :
- elle présente des extrémités libres axiales opposées (67a, 67b) et une longueur entre ces extrémités, et
- le ou au moins certains des fils souples (57) s'étend sur l'essentiel au moins de ladite longueur de la prothèse.

7. Prothèse selon l'une quelconque des revendications précédentes, **caractérisée en ce que** le fil souple ou au moins un parmi les fils souples est noué au moins une fois sur sa longueur.

## Patentansprüche

1. Medizinische Prothese, die geeignet ist, in einen Gang eines lebenden Körpers implantiert zu werden, und die aufweist:
- eine Armierung (1, 10), die durch einen oder mehrere röhrenförmige Abschnitte (13, 15, 17, 19; 27, 29, 31) entlang mindestens einer Rohrachse (21, 55, 37) bestimmt ist, wobei der oder mindestens einige der Abschnitte einen Mäanderaufbau aufweist, der eine Steifigkeit hat und Scheitel (11 ) aufweist, zwischen denen sich längliche Segmente (12, 14) erstrecken, wobei die Mäander des oder der röhrenförmigen Abschnitte, einem oder mehreren Einrollniveaus (17a, 19a; 47, 49, 51) folgend, eingerollt sind,
- eine flexible Hülle (54), die zu der Armierung im wesentlichen koaxial ist, um hier ein in dem Gang des Körpers zirkulierendes Fluid zu kanalisieren,
- und Verbindungsmittel (57, 57a, 57b, ..., 57e) zwischen der Hülle und der Armierung, wobei diese Verbindungsmittel mindestens einen flexiblen Draht aufweisen, dessen Steifigkeit geringer ist als die der Segmente des Aufbaus, wobei der flexible Draht (57, 57a, ..., 57e) angeordnet ist, um diese Verbindung derart zu si-, chern, daß er mindestens ein Segment überlappt und in die Hülle geht,
- wobei die Armierung mehrere röhrenförmige Abschnitte (13, 15, ...) aufweist,
**dadurch gekennzeichnet, daß** sich der oder mindestens einer der flexiblen Drähte mit solcher Anordnung nacheinander entlang mindestens zweier Aufbausegmente (14; 14a, 14b, 12) erstreckt, die zu unterschiedlichen röhrenförmigen Abschnitten gehören.

2. Prothese nach Anspruch 1, **dadurch gekennzeichnet, daß** der flexible Draht von einem Gang eines Segmentes zu dem anderen in der Nähe oder um einen ihrer Scheitel (11 ) geht, um das Beibehalten dieses Scheitels zu begünstigen, wenn die Prothese an dieser Stelle gekrümmt wird.

3. Prothese nach Anspruch 1 oder Anspruch 2, **dadurch gekennzeichnet, daß** der betreffende flexible Draht im wesentlichen als Schraube einem Vorschiebepunkt folgend um mindestens zwei Segmente eingerollt ist.

4. Prothese nach Anspruch 2 oder Anspruch 3, **dadurch gekennzeichnet, daß** die mindestens zwei Segmente im wesentlichen in Kontinuität zueinander, einer im wesentlichen geraden oder schraubenförmigen Richtung folgend, angeordnet sind, wobei sich der betreffende flexible Draht so in allgemeiner Weise, dieser Richtung folgend, erstreckt.

5. Prothese nach einem der vorhergehenden Ansprüche, **dadurch gekennzeichnet, daß** sie mehrere flexible Drähte (57, 57c, ...) aufweist, die sich mehreren zueinander parallel verlaufenden Richtungen folgend erstrecken.

6. Prothese nach einem der vorhergehenden Ansprüche, **dadurch gekennzeichnet, daß**:
- sie axial gegenüberliegende freie Enden (67a, 67b) und eine Länge zwischen diesen Enden aufweist, und
- sich der oder mindestens einige der flexiblen Drähte (57) auf dem Hauptteil mindestens dieser Länge der Prothese erstrecken.

7. Prothese nach einem der vorhergehenden Ansprüche, **dadurch gekennzeichnet, daß** der flexible Draht oder mindestens einer der flexiblen Drähte mindestens einmal auf seiner Länge geknotet ist.

## Claims

1. A medical prosthesis adapted to be implanted into a duct of a living body, comprising:
- a frame (1, 10) defined by one or several tubular sections (13, 15, 17, 19 ; 27, 29, 31) extending along at least one tubular axis (21, 55, 37), the section or at least some tubular sections comprising a meandering structure having a rigidity and presenting apices (11) between which elongate segments (12, 14) extend, the meanders of the section or said tubular sections being rolled up at one or more rolling levels (17a, 19a ; 47, 49, 51),
- a flexible sleeve (54) which is substantially coaxial with the frame, to channel therein a fluid which is circulating in the duct of said body, and
- means (57, 57a, 57b, ..., 57e) for connecting the sleeve and the frame, said connecting means including at least one flexible thread of a rigidity which is less than that of the segments of the structure, said flexible thread (57, 57a, ..., 57e) being arranged for ensuring said connection, to overlap at least one segment and to pass into the sleeve,
- the frame presenting several tubular sections (13, 15, ...),
**characterized in that**:
the thread or at least one of flexible threads, so arranged, extends successively along at least two segments of structure (14 ; 14a, 14b, 12) included into different tubular sections.

2. Prosthesis according to claim 1, **characterized in that** the flexible thread passes, in the course of passing from one segment to another, close about or around one of the apices (11) thereof for improving the retention of said apex when the prosthesis is curved there.

3. Prosthesis according to claim 1 or claim 2, **characterized in that** said flexible thread is substantially helically wound about said at least two segments, as a straight stitch.

4. Prosthesis according to claim 2 or claim 3 **characterized in that** said at least two segments are substantially situated so as to be a continuation of one another, along a substantially rectilinear or helical direction, so that said flexible thread generally extends along said direction.

5. Prosthesis according to any of the previous claims, **characterized in that** it comprises several flexible threads (57, 57c,...) which extend along several directions which are substantially parallel to one another.

6. Prosthesis according to any of the previous claims, **characterized in that**:
- it has opposite axial free ends (67a, 67b) and a length therebetween and;
- the thread or some of flexible threads (57) extend at least over the main part of the length of the prosthesis.

7. Prosthesis according to anyone of preceding claims, **characterized in that** the flexible thread or at least one of the flexible threads has at least one knot along its length.
